# FASCICULE DE BREVET EUROPEEN

(11) **EP 2 395 998 B1**
(45) Date de publication et mention de la délivrance du brevet: **08.07.2015**
(21) Numéro de dépôt: 10709876.6
(22) Date de dépôt: 16.02.2010
(51) Int. Cl.: A61K 31/4164, A61K 31/498, A61K 31/7048, A61K 45/06

(54) **ASSOCIATION DE L'IVERMECTINE AVEC LA BRIMONIDINE POUR LE TRAITEMENT OU LA PRÉVENTION DE LA ROSACEE**
KOMBINATION AUS IVERMECTIN MIT BRIMONIDIN ZUR BEHANDLUNG ODER PRÄVENTION VON ROSAZEA
COMBINATION OF IVERMECTIN WITH BRIMONIDINE FOR TREATING OR PREVENTING ROSACEA

(30) Priorité: 16.02.2009 FR 0950981
(43) Date de publication de la demande: 21.12.2011
(73) Titulaire: Galderma Research & Development, 06410 Biot (FR)
(72) Inventeur: JOMARD, André, 06460 Saint Vallier De Thiey (FR); FREDON, Laurent, 06330 Roquefort Les Pins (FR); ROYE, Olivier, 83440 Fayence (FR)
(74) Mandataire: Gallois, Valérie
(86) Numéro de dépôt international: PCT/FR2010/050259
(87) Numéro de publication internationale: WO 2010/092312

(56) Documents cités:
- WO-A-2006/097628
- WO-A-2006/131651
- WO-A-2006/131652
- WO-A-2006/131653
- WO-A-2007/054822
- WO-A-2008/037935
- WO-A-2008/037936
- FR-A- 2 901 703
- US-A1- 2005 020 600
- US-A1- 2005 165 079
- ASIO S M ET AL: "Mansonella perstans: safety and efficacy of ivermectin alone, albendazole alone and the two drugs in combination." ANNALS OF TROPICAL MEDICINE AND PARASITOLOGY JAN 2009, vol. 103, no. 1, janvier 2009 (2009-01), pages 31-37, XP009122669 ISSN: 0003-4983

## Description

L'invention est limitée à l'objet défini dans les présentes revendications; la description ci-après est sujette à cette limitation.

L'invention concerne une association de composés pour le traitement de d'affections dermatologiques chez l'homme, particulièrement la rosacée et la rosacée oculaire.

La rosacée est une dermatose inflammatoire commune chronique et progressive liée à une relaxation vasculaire. Elle affecte principalement la partie centrale du visage et se caractérise par un rougissement du visage ou des bouffées de chaleur, un érythème facial, des papules, des pustules, une télangiectasie et parfois des lésions oculaires appelées rosacée oculaire. Dans des cas graves, particulièrement chez l'homme, le tissu mou du nez peut enfler et produire un gonflement bulbeux appelé rhinophyma.

La rosacée survient généralement entre l'âge de 25 et 70 ans, et elle est beaucoup plus commune chez les gens au teint clair. Elle touche plus particulièrement les femmes, bien que cette affection soit généralement plus sévère chez l'homme. La rosacée est chronique et persiste des années avec des périodes d'exacerbation et de rémission.

La pathogenèse de la rosacée est mal connue. De nombreux facteurs peuvent être impliqués sans forcément induire cette affection. Ce sont par exemple des facteurs psychologiques, des troubles gastro-intestinaux, des facteurs environnementaux (exposition au soleil, température, humidité), émotionnels (stress), alimentaires (alcool, épices), hormonaux, vasculaires, voire une infection par *Helicobacter pilori.*

Classiquement, la rosacée est traitée oralement ou topiquement par des antibiotiques tels que les tétracyclines, l'érythromycine, la clindamycine, mais aussi par la vitamine A, l'acide salicylique, des agents antifongiques, des stéroïdes, le métronidazole (un agent antibactérien) ou par l'isotrétinoïne dans les formes sévères ou encore par des anti-infectieux tel que le peroxyde de benzoyle ou encore par l'acide azélaique. On connaît également le traitement de la rosacée avec de l'ivermectine qui cible le parasite *Demodex folliculorum* présent sur la peau des patients (US 5,952,372). On connaît également le traitement de la rosacée par des agonistes des récepteurs adrénergiques alpha-1 ou alpha-2 (US 2006/0171974A1, US 2005/0165079A1, US 2005/0020600A1).

Ces traitements présentent des effets secondaires désagréables pour le patient tels des phénomènes d'irritation ou d'intolérance. De plus, aucun des traitements existants ne permettent de traiter et/ou prévenir efficacement l'ensemble des symptômes associés à la rosacée.

Tenant compte de ce qui précède, il existe donc un besoin de réaliser un traitement plus efficace de la rosacée, qui ne présente pas les effets secondaires observés dans l'art antérieur. Il existe notamment un besoin de réaliser une composition conférant une plus grande tolérance des principes actifs, tout en diminuant leurs effets secondaires

De manière surprenante, la Demanderesse a observé qu'une association d'un composé de la famille des avermectines ou de la famille des mylbemycines et d'un composé de la famille des agonistes des récepteurs adrénergiques alpha-1 ou alpha-2 permet un traitement plus efficace de la rosacée, avec moins d'effets secondaires quelle que soit la durée d'application de cette association. En particulier, une telle association permet de réduire sensiblement la durée du traitement et d'obtenir une réduction plus importante des symptômes de la rosacée. Cette association peut permettre en outre de supprimer l'effet rebond habituellement observé en fin de traitement avec les agonistes des récepteurs adrénergiques alpha-1 ou alpha-2.

L'invention a pour objet une association d'un composé de la famille des avermectines ou de la famille des mylbemycines et d'un composé de la famille des agonistes des récepteurs adrénergiques alpha-1 ou alpha-2 pour son application comme médicament dans le traitement et/ou la prévention des affections dermatologiques et particulièrement la rosacée et la rosacée oculaire.

L'invention a également pour objet l'utilisation d'une association d'un composé de la famille des avermectines ou de la famille des mylbemycines et d'un composé de la famille des agonistes des récepteurs adrénergiques alpha-1 ou alpha-2 pour la fabrication d'un médicament destiné au traitement et/ou la prévention des affections dermatologiques et particulièrement la rosacée et la rosacée oculaire.

La présente invention a également pour objet une composition pharmaceutique, notamment dermatologique, comprenant, dans un milieu physiologiquement acceptable, au moins un composé de la famille des avermectines ou de la famille des mylbemycines et au moins un composé de la famille des agonistes des récepteurs adrénergiques alpha-1 ou alpha-2 destinée au traitement et/ou la prévention des affections dermatologiques et particulièrement la rosacée et la rosacée oculaire.

Par composition dermatologique, on entend une composition pharmaceutique appliquée sur la peau.
Par milieu physiologiquement acceptable, on entend un milieu compatible avec la peau, les muqueuses et/ou les phanères.

Par affections dermatologiques, on entend des désordres cutanés et oculaire. On peut citer comme exemple non limitatif l'acné, l'hyperséborrhée, la rosacée, la rosacée oculaire, le psoriasis, la dermite atopique.
L'affection dermatologique est plus particulièrement la rosacée ou la rosacée oculaire.

L'invention a également pour objet l'utilisation d'une telle composition pour la fabrication d'un médicament destiné à la prévention et/ou au traitement des affections dermatologiques et particulièrement la rosacée et la rosacée oculaire.

L'invention a également pour objet un produit sous forme de kit contenant :
(a) une première composition comprenant un composé de la famille des avermectines ou de la famille des mylbemycines, et
(b) une seconde composition, distincte de la première, comprenant un composé de la famille des agonistes des récepteurs adrénergiques alpha-1 ou alpha-2,
comme produit de combinaison pour son application comme médicament dans le traitement et/ou la prévention des affections dermatologiques et particulièrement la rosacée et la rosacée oculaire, lesdites première et seconde compositions pouvant être appliquées de manière simultanée, séparée ou étalée dans le temps.

L'invention a également pour objet l'utilisation d'un produit sous forme de kit contenant :
(a) une première composition comprenant un composé de la famille des avermectines ou de la famille des mylbemycines, et
(b) une seconde composition, distincte de la première, comprenant un composé de la famille des agonistes des récepteurs adrénergiques alpha-1 ou alpha-2,

comme produit de combinaison pour la fabrication d'un médicament destiné au traitement et/ou la prévention des affections dermatologiques et particulièrement la rosacée et la rosacée oculaire, lesdites première et seconde composition pouvant être appliquées de manière simultanée, séparée ou étalée dans le temps.

Selon l'invention, le composé de la famille des avermectines est avantageusement choisi parmi l'ivermectine, l'invermectine, l'avermectine, l'abamectine, la doramectine, l'eprinomectine et la sélamectine, l'aversectine B, AB ou C, l'émamectine B1a, l'émamectine B1b et leurs dérivés, ou la latidectine . Le composé de la famille des avermectines est de préférence l'ivermectine.

Selon l'invention, le composé de la famille des mylbemycines est avantageusement choisi parmi la lépimectine, la milbemectine, l'oxime de milbemycine, la moxidectine, la 6'-éthyl-lepimectine, la 6'-méthyl-lépimectine et ses dérivés ou la némadectine α, β, γ ou δ.

Selon l'invention, le composé de la famille des agonistes des récepteurs adrénergiques alpha-1 est avantageusement choisi parmi le metaraminol bi-tartrate, midodrine, methoxamine, mephentermine, phenylephrine, oxymetazoline, la tetrahydrozoline, la naphazoline ou la xylometazoline ou leurs sels.

Plus particulièrement, le composé de la famille des agonistes des récepteurs adrénergiques alpha-1 tel que défini ci-dessus est sous forme de chlorhydrate ou de bi-tartrate.

Selon l'invention, le composé de la famille des agonistes des récepteurs adrénergiques alpha-2 est avantageusement choisi parmi l'apraclonidine, la brimonidine, la clonidine, mirtazapine, dexmedetomidine, guanbenz acetate, lidamidine, lofexidine, methyldopa, rilmenidine, talipexole, tiamenidine, tizanidine, la tolonidine ou leurs sels.

Plus particulièrement, le composé de la famille des agonistes des récepteurs adrénergiques alpha-2 tel que défini ci-dessus est sous forme de tartrate.

Plus particulièrement le composé de la famille des agonistes des récepteurs adrénergiques alpha-2 peut être la brimonidine ou son sel tartrique.

L'association selon l'invention contient plus particulièrement un composé de la famille des avermectines et un composés de la famille des agonistes des récepteurs adrénergiques alpha 2.

Préférentiellement, l'association selon l'invention contient de la brimonidine et de l'ivermectine.

Dans le cadre de la présente invention, une association d'un composé de la famille des avermectines ou de la famille des mylbemycines avec un composé de la famille des agonistes des récepteurs adrénergiques alpha-1 ou alpha-2, signifie que lesdits composés associés peuvent être soit présents au sein d'une même composition, soit présents séparément l'un de l'autre au sein de compositions distinctes, formant par exemple un produit sous forme de kit. En d'autres termes, ces composés sont destinés à être administrés à un patient dans le cadre d'un même traitement, c'est-à-dire sur une période commune de traitement, soit en même temps, en étant compris ou pas au sein d'une seule et même composition, soit en des instants différents. De plus, ils peuvent être administrés par des modes d'administration identiques ou différents et/ou être compris dans des compositions identiques ou différentes.

L'association des composés précités présents séparément au sein de compositions distinctes, et notamment dans le cas d'un produit sous forme de kit, permet de limiter les interactions du ou des composés de la famille des avermectines, notamment l'ivermectine, ou de la famille des mylbemycines, avec le ou les composés de la famille des agonistes des récepteurs adrénergiques alpha-1 ou alpha-2. Cela permet également de limiter au maximum les interactions du ou des composés de la famille des avermectines, notamment l'ivermectine, ou de la famille des mylbemycines, avec les nombreux excipients habituellement contenus dans une composition unique, et notamment les excipients contenus dans la composition comprenant les composés de la famille des agonistes des récepteurs adrénergiques alpha-1 ou alpha-2. Les compositions selon l'invention, appliquées simultanément ou successivement, sont ainsi très bien tolérées, précises en terme de quantité de composés actifs délivrés, et d'utilisation pratique. Elles offrent en outre confort et hydratation aux patients.

Dans le cas d'une association des composés précités présents séparément au sein de compositions distinctes, et notamment dans le cas d'un produit sous forme de kit, un composé de la famille des avermectines ou de la famille des mylbemycines peut être d'abord appliqué sur la peau d'un patient, puis un composé de la famille des agonistes des récepteurs adrénergiques alpha-1 ou alpha-2 peut être appliqué, ou inversement.

Dans les compositions selon l'invention, le composé de la famille des avermectines ou de la famille des mylbemycines est présent à une concentration comprise entre 0,001 et 10 % en poids, par rapport au poids total de la composition le comprenant, de préférence entre 0,01 et 5 % en poids, et en particulier 0,75%, 1%, 1,5% ou 2%. Lorsqu'une composition comprend plusieurs de ces composés, leur concentration totale est comprise dans les quantités précitées.

Dans les compositions selon l'invention, le composé de la famille des agonistes des récepteurs adrénergiques alpha-1 ou alpha-2 est présent à une concentration comprise entre 0,01 et 20 % en poids par rapport au poids total de la composition, de préférence entre 0,02 et 10 %, de manière particulièrement préférée entre 0,05 et 5 % en poids par rapport au poids total de la composition. Lorsqu'une composition comprend plusieurs de ces composés, leur concentration totale est comprise dans les quantités précitées.

De façon particulièrement préférée, l'association comprend un composé de la famille des avermectines ou de la famille des mylbemycines présent à une concentration comprise entre 0,01 et 5 % en poids, par rapport au poids total de la composition le comprenant, et un composé de la famille des agonistes des récepteurs adrénergiques alpha-1 ou alpha-2 présent à une concentration comprise entre 0,01 et 5 % en poids par rapport au poids total de la composition.

Ladite composition selon l'invention contient plus particulièrement un composé de la famille des avermectines et un composés de la famille des agonistes des récepteurs adrénergiques alpha 2.

Préférentiellement, la composition selon l'invention comprend de la brimonidine et de l'ivermectine.

L'association selon l'invention et les compositions comprenant les composés de cette association sont notamment destinées à une application topique sur la peau et/ou à une application oculaire sur les yeux.

Les compositions de l'invention comprennent en outre un véhicule pharmaceutiquement ou cosmétiquement acceptable, c'est-à-dire un véhicule adapté pour une utilisation en contact avec des cellules humaines, sans toxicité, irritation, réponse allergique indue et similaire, et proportionné à un rapport avantage/risque raisonnable.

Les compositions de l'invention peuvent comprendre, en outre, au moins un autre agent thérapeutique susceptible d'augmenter l'efficacité du traitement.

Les compositions de l'invention peuvent comprendre en outre tout additif usuellement utilisé dans le domaine pharmaceutique, dermatologique, compatible avec le composé de la famille des avermectines ou de la famille des mylbemycines et/ou le composé de la famille des agonistes des récepteurs adrénergiques alpha-1 ou alpha-2 en présence.

On peut citer notamment des séquestrants, des antioxydants, des filtres solaires, des conservateurs, par exemple la DL-alpha-tocophérol, des charges, des électrolytes, des humectants, des colorants, de bases ou d'acides usuels, minéraux ou organiques, des parfums, des huiles essentielles, des actifs cosmétiques, des hydratants, des vitamines, des acides gras essentiels, des sphingolipides, des composés autobronzants tels que la DHA, des agents apaisants et protecteurs de la peau tels que l'allantoïne, des agents propénétrants, des gélifiants ou un mélange de ceux-ci. Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées.

Ces additifs peuvent être présents dans la composition à raison de 0 à 20 % en poids par rapport au poids total de la composition.

L'administration peut être effectuée par voie topique, entérale ou orale, parentérale ou oculaire.

Parmi ces voies d'administration, la voie topique et la voie oculaire sont particulièrement préférées.

Les compositions de la présente invention peuvent se présenter sous toutes les formes galéniques normalement utilisées pour une application topique, notamment sous forme de solutions, de lotion, de gels, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-liquide ou solide du type crème, pommade ou encore de micro-émulsions, de micro-capsules, de micro-particules ou de dispersions vésiculaires de type ionique et/ou non ionique.

De manière avantageuse, la composition comprend une pommade, une crème, une lotion ou un gel.

Il va être maintenant donné, à titre d'illustration et sans aucun caractère limitatif, diverses formulations de compositions selon l'invention ainsi que les résultats d'étude de l'activité anti-inflammatoire de l'association d'un composé de la famille des avermectines ou de la famille des mylbemycines et d'un composé de la famille des agonistes des récepteurs adrénergiques alpha-1 ou alpha-2.

### EXEMPLE 1

| Ingrédients | % en poids par rapport au poids total de la composition |
|---|---|
| Ivermectine | 1.00 |
| Brimonidine tartrate | 0.20 |
| EDTA | 0.1 |
| Polysorbate 80 | 8.0 |
| Propylene Glycol | 20.00 |
| Alcool benzylique | 3 |
| Eau | Qsp 100 |

### EXEMPLE 2 ne fait pas partie de l'invention

| Ingrédients | % en poids par rapport au poids total de la composition |
|---|---|
| Emamectine | 0.5 |
| Brimonidine tartrate | 0.3 |
| Vaseline Codex | 56.00 |
| Huile de vaseline | 43.00 |

### EXEMPLE 3 ne fait pas partie de l'invention

| Ingrédients | % en poids par rapport au poids total de la composition |
|---|---|
| Ivermectin | 1.40 |
| Chlorhydrate d'oxymetazoline | 0.20 |
| Glycérine | 4.0 |
| Steareth-2 | 1.0 |
| Steareth-21 | 2.0 |
| Silicate d'aluminium et de magnésium/ dioxyde de titane/silice | 1.0 |
| Parahydroxybenzoate de méthyle | 0.2 |
| Parahydroxybenzoate de propyle | 0.1 |
| EDTA disodique | 0.05 |
| Acide Citrique monohydrate | 0.05 |
| Palmitate d'Isopropyle | 4.0 |
| Glyceryle/PEG 100 stéarate | 2.0 |
| Cire autoémulsionnable | 1.0 |
| Acide palmitostéarique | 2.00 |
| Dimethicone 200-350 cS | 0.5 |
| Propylene Glycol | 4.0 |
| Triacetate de glycérol | 1.00 |
| Phenoxyethanol | 0.5 |
| Hydroxyde de Sodium 10% | Qs pH |
| Eau | Qsp 100 |

### EXEMPLE 4

| Ingrédients | % en poids par rapport au poids total de la composition |
|---|---|
| Ivermectine | 0.03 |
| Brimonidine | 0.15 |
| Polysorbate 80 | 2.00 |
| Chlorure de Benzalkonium | 0.05 |
| EDTA | 0.05 |
| Eau | qsp 100 |
| Système tampon | pH 6.3 |

### EXEMPLE 5: Evaluation de l'activité anti-inflammatoire de l'ivermectine et de la brimonidine après une application topique unique dans le test de l'oedème de l'oreille de souris induit par l'acide arachidonique sur les souris Balb/c

L'acide arachidonique est dissous dans un mélange THF/Methanol à 4%.

### Traitement:

L'ivermectine est dissous dans la solution d'acide arachidonique (AA) et testé à la concentration de 1%.
20µl de la solution sont appliqués sur la face interne de l'oreille droite.

L'épaisseur de l'oreille est mesurée à T+1h T+2h et T+4h.

### Résultats :

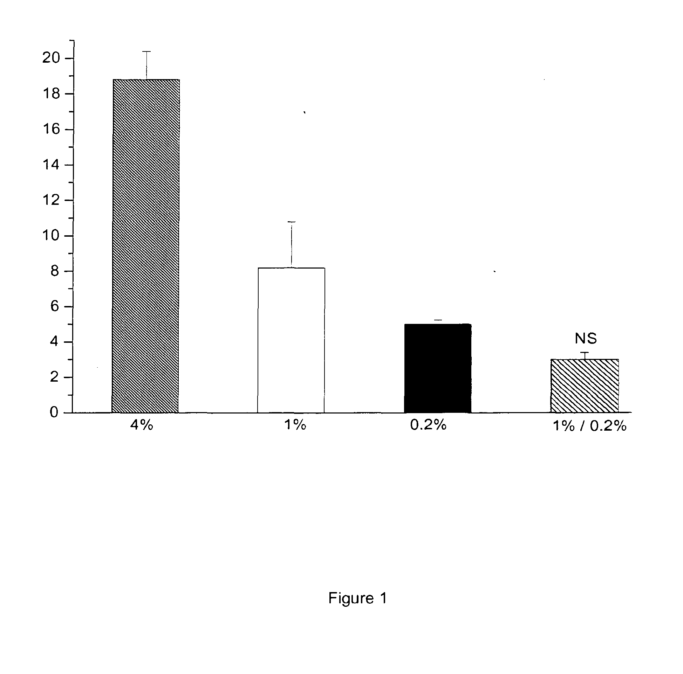

La figure 1 représente la mesure moyenne de l'épaisseur de l'oedème de l'oreille, c'est-à-dire la mesure moyenne de l'épaisseur de l'oreille après traitement à laquelle on soustrait la mesure moyenne de l'épaisseur de l'oreille obtenue avec le groupe témoin (non traité). Et ceci après traitement à l'acide arachidonique à 4% (▧), l'ivermectine à 1% (□), la brimonidine à 0.2% (▩), l'association de l'ivermectine et de la brimonidine (▧).

L'indométacine (contrôle positif) à 5% inhibe l'oedème de l'oreille dû à l'acide arachidonique de 95% (***).
L'ivermectine seul (1%) réduit l'oedème de l'oreille de 56% (**).
L'association ivermectine (1%) avec la brimonidine (0.2%) inhibe l'oedème de l'oreille dû à l'acide arachidonique de 84% (***), elle montre donc un fort effet anti-inflammatoire dans le modèle de l'oedème de l'oreille de souris induit par l'acide arachidonique.

### EXEMPLE 6 : Evaluation de l'activité anti-inflammatoire de l'ivermectine et de la brimonidine après une application topique unique dans le test de l'oedème de l'oreille de souris induit par le TPA sur les souris Balb/c

### Traitement :

L'oedème est induit par application unique de 20µl de TPA (forbol-12-myristate-13 acetate) dissous dans l'éthanol à 0.01%.
Les composés à tester sont dilués dans la solution de TPA.
Un contrôle positif, le valérate de β-méthasone (VdB) à 0.01% est également testé, il inhibe l'oedème de l'oreille de souris de 89%.

L'ivermectine est appliqué à une concentration de 0.1%, 0.3% et 1%. La brimonidine est ajoutée à la concentration de 0.2%.
L'épaisseur de l'oreille de souris est mesurée à T+6h.

### Résultats :

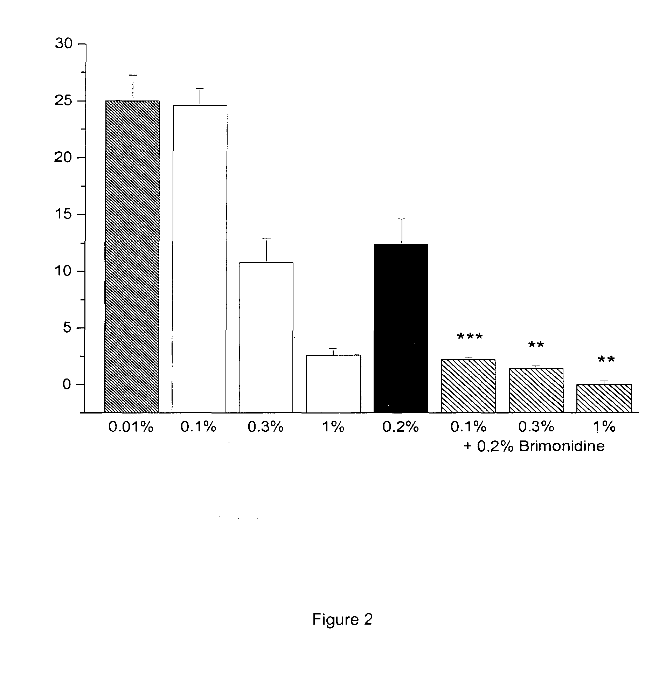

Les résultats sont présentés en figure 2.
La figure 2 représente la mesure moyenne de l'épaisseur de l'oedème de l'oreille en fonction de la dose de composé à tester en %, c'est-à-dire la mesure moyenne de l'épaisseur de l'oreille après traitement à laquelle on soustrait la mesure moyenne de l'épaisseur de l'oreille obtenue avec le groupe témoin (non traité).
Et ceci après traitement au TPA à 0.01 % (▧),
l'ivermectine à respectivement à 0.1, 0.3 et 1% (□),
la brimonidine à 0.2% (▩),
l'association de l'ivermectine et de la brimonidine (▧).

Après une application unique topique de l'ivermectine à 0.3% et 1% dilué dans la solution de TPA, une réduction de l'oedème de l'oreille respectivement de 57% (**) et 90% (***) est observée.
L'application de la brimonidine seule à 0.2% réduit l'oedème de l'oreille de souris de 50%.
L'association de l'ivermectine (à 0.1, 0.3 et 1%) et de la Brimonidine (0.2%) a un effet anti-inflammatoire dose-dépendant, et réduit l'oedème de l'oreille induit par le TPA respectivement de 91% (***) (à 0.1%), 94 %(***) (à 0.3%) et 100% (***) (à 1%).

## Revendications

1. Association de l'ivermectine et de la brimonidine ou ses sels, pour son application comme médicament dans le traitement et/ou la prévention de la rosacée et/ou de la rosacée oculaire.

2. Association selon la revendication 1, **caractérisée en ce que** l'ivermectine et la brimonidine ou ses sels sont présents au sein d'une même composition.

3. Association selon la revendication 1, **caractérisée en ce que** l'ivermectine et la brimonidine ou ses sels sont présents séparément l'un de l'autre au sein de compositions distinctes.

4. Association selon l'une quelconque des revendications précédentes, **caractérisée en ce que** l'ivermectine est présente à une concentration comprise entre 0,001 et 10 % en poids, par rapport au poids total de la composition la comprenant, et **en ce que** la brimonidine ou ses sels sont présents à une concentration comprise entre 0,01 et 20 % en poids par rapport au poids total de la composition.

5. Utilisation d'une association telle que définie dans l'une des revendications précédentes, pour la fabrication d'un médicament destiné au traitement et/ou la prévention de la rosacée et/ou de la rosacée oculaire.

6. Produit sous forme de kit contenant :
(a) une première composition comprenant l'ivermectine, et
(b) une seconde composition, distincte de la première, comprenant la brimonidine ou ses sels,
comme produit de combinaison pour son application comme médicament dans le traitement et/ou la prévention de la rosacé et de la rosacée oculaire, lesdites première et seconde composition pouvant être appliquées de manière simultanée, séparée ou étalée dans le temps.

7. Utilisation d'un produit tel que défini selon la revendication 6 pour la fabrication d'un médicament destiné au traitement et/ou la prévention de la rosacée et/ou de la rosacée oculaire.

8. Composition pharmaceutique, notamment dermatologique, comprenant, dans un milieu physiologiquement acceptable, au moins de l'ivermectine, et au moins de la brimonidine ou ses sels.

9. Composition selon la revendication 8, **caractérisée en ce que** l'ivermectine représente entre 0,001 et 10 % en poids, par rapport au poids total de la composition, de préférence entre 0,01 et 5 % en poids.

10. Composition selon la revendication 8, **caractérisée en ce que** la concentration de la brimonidine ou ses sels est comprise entre 0,01 et 20 % en poids, par rapport au poids total de la composition, de préférence entre 0.02 et 10 % en poids, par rapport au poids total de la composition.

11. Composition selon l'une quelconque des revendications 8 à 10, **caractérisée en ce qu'**elle est d'application topique.

12. Composition selon l'une quelconque des revendications 8 à 10, **caractérisée en ce qu'**elle est d'application oculaire.

## Patentansprüche

1. Kombination aus Ivermectin und Brimonidin oder seiner Salze für die Applikation als Arzneimittel bei der Behandlung und/oder Prävention der Rosazea und/oder der okulären Rosazea.

2. Kombination gemäß Anspruch 1, **dadurch gekennzeichnet, dass** Ivermectin und Brimonidine oder seine Salze in derselben Zusammensetzung vorliegen.

3. Kombination gemäß Anspruch 1, **dadurch gekennzeichnet, dass** Ivermectin und Brimonidine oder seine Salze getrennt voneinander in verschiedenen Zusammensetzungen vorliegen.

4. Kombination gemäß einem der vorhergehenden Ansprüche, **dadurch gekennzeichnet, dass** Ivermectin mit einer Konzentration zwischen 0,001 und 10 Gew.-% bezogen auf das Gesamtgewicht der Ivermectin-haltigen Zusammensetzung vorliegt und dass Brimonidin oder seine Salze mit einer Konzentration zwischen 0,01 und 20 Gew.-% bezogen auf das Gesamtgewicht der Zusammensetzung vorliegen.

5. Verwendung einer Kombination wie in einem der vorhergehenden Ansprüche definiert zur Herstellung eines Arzneimittels für die Behandlung und/oder Prävention der Rosazea und/oder der okulären Rosazea.

6. Produkt in Form eines Kits, umfassend:
(a) eine erste Zusammensetzung, umfassend Ivermectin, und
(b) eine von der ersten getrennte zweite Zusammensetzung, umfassend Brimonidin oder seine Salze,
als Kombinationsprodukt für die Applikation als Arzneimittel bei der Behandlung und/oder Prävention der Rosazea und der okulären Rosazea, wobei besagte erste und zweite Zusammensetzung gleichzeitig, getrennt oder über die Zeit verteilt appliziert werden können.

7. Verwendung eines Produkts wie gemäß Anspruch 6 definiert zur Herstellung eines Arzneimittels für die Behandlung und/oder Prävention der Rosazea und/oder der okulären Rosazea.

8. Pharmazeutische Zusammensetzung, insbesondere dermatologische Zusammensetzung, umfassend in einem physiologisch verträglichen Milieu wenigstens Ivermectin und wenigstens Brimonidin oder seine Salze.

9. Zusammensetzung gemäß Anspruch 8, **dadurch gekennzeichnet, dass** Ivermectin zwischen 0,001 und 10 Gew.-%, vorzugsweise zwischen 0,01 und 5 Gew.-%, bezogen auf das Gesamtgewicht der Zusammensetzung umfasst.

10. Zusammensetzung gemäß Anspruch 8, **dadurch gekennzeichnet, dass** die Konzentration von Brimonidin oder seiner Salze zwischen 0,01 und 20 Gew.-% bezogen auf das Gesamtgewicht der Zusammensetzung, vorzugsweise zwischen 0,02 und 10 Gew.-% bezogen auf das Gesamtgewicht der Zusammensetzung, umfasst.

11. Zusammensetzung gemäß einem der Ansprüche 8 bis 10, **dadurch gekennzeichnet, dass** sie topisch appliziert wird.

12. Zusammensetzung gemäß einem der Ansprüche 8 bis 10, **dadurch gekennzeichnet, dass** sie okulär appliziert wird.

## Claims

1. A combination of ivermectin and brimonidine or salts thereof, for application as a medicament for treating and/or preventing rosacea or ocular rosacea.

2. The combination according to claim 1, wherein ivermectin and brimonidine or salts thereof are present within a common composition.

3. The combination according to claim 1, wherein ivermectin and brimonidine or salts thereof are present separately one to the other within distinct compositions.

4. The combination according to any one of the preceding claims, wherein ivermectin is present in an amount of between 0.001 and 10% by weight, relative to the total weight of the composition comprising it, and wherein brimonidine or salts thereof are present in an amount of between 0.01 and 20% by weight relative to the total weight of the composition.

5. The use of a combination according to one of the preceding claims, for the production of a medicament intended for treating and/or preventing rosacea and/or ocular rosacea.

6. A product in the form of a kit containing:
(a) a first composition comprising ivermectin, and
(b) a second composition, distinct from the first one, comprising brimonidine or salts thereof, as a combination product for application thereof as a medicament for treating and/or preventing rosacea and ocular rosacea, wherein said first and second compositions can be applied simultaneously, separately or with a time delay.

7. The use of a product as defined according to claim 6 for the production of a medicament intended for treating and/or preventing rosacea and/or ocular rosacea.

8. A pharmaceutical, in particular dermatological composition, comprising, in a physiologically acceptable medium, at least ivermectin, and at least brimonidine or salts thereof.

9. The composition according to claim 8, wherein ivermectin represents between 0.001 and 10% by weight, relative to the total weight of the composition, preferably between 0.01 and 5% by weight.

10. The composition according to claim 8, wherein the concentration of brimonidine or salts thereof is between 0.01 and 20% by weight, relative to the total weight of the composition, preferably between 0.02 and 10% by weight, relative to the total weight of the composition.

11. The composition according to any of claims 8 to 10, wherein it is for topical application.

12. The composition according to any of claims 8 to 10, wherein it is for ocular application.
